# EUROPÄISCHE PATENTSCHRIFT

(11) **EP 3 855 583 B1**
(45) Veröffentlichungstag und Bekanntmachung des Hinweises auf die Patenterteilung: **10.05.2023**
(21) Anmeldenummer: 21151057.3
(22) Anmeldetag: 12.01.2021
(51) Int. Cl.: H01R 24/20, H01R 24/28, H01R 24/00, H01R 13/64, A61M 16/10, A61M 16/08, A61M 16/16, H01R 13/46

(54) **ELEKTRISCHE STECKVERBINDUNG FÜR EINE MEDIZINGERÄTEANORDNUNG**
ELECTRICAL PLUG CONNECTION FOR A MEDICAL DEVICE ASSEMBLY
CONNECTEUR ENFICHABLE ÉLECTRIQUE POUR UN AGENCEMENT D'APPAREIL MÉDICAL

(30) Priorität: 24.01.2020 DE 102020000422
(43) Veröffentlichungstag der Anmeldung: 28.07.2021
(73) Patentinhaber: Drägerwerk AG & Co. KGaA, 23558 Lübeck (DE)
(72) Erfinder: Hampe, Markus, 23558 Lübeck (DE); Kämer, Markus, 23558 Lübeck (DE)

(56) Entgegenhaltungen:
- WO-A1-2019/147774
- US-A1- 2019 344 037

## Beschreibung

Die Erfindung betrifft ein elektrisches Steckverbindungssystem mit mindestens zwei erfindungsgemäßen elektrischen Steckverbindungen sowie eine Medizingeräteanordnung mit dem erfindungsgemäßen elektrischen Steckverbindungssystem.

Elektrische Steckverbindungen sind in verschiedenen Formen und für verschiedene Anwendungen bekannt. Typischerweise besteht eine elektrische Steckverbindung aus einem Steckelement, das den männlichen Teil der elektrischen Steckverbindung bildet und aus einem Aufnahmeelement, das den weiblichen Teil der elektrischen Steckverbindung bildet. Über entsprechende Kontaktbereiche an den beiden Elementen wird durch die elektrische Steckverbindung eine elektrische Verbindung zwischen den beiden Elementen bereitgestellt.

Weiterhin ist bekannt, Steckelement und Aufnahmeelement jeweils spiegelsymmetrisch zu gestalten, um eine Umpolung der Steckverbindung zu ermöglichen und eine Handhabung der Steckverbindung im Alltag zu vereinfachen. Insbesondere für den haushaltsüblichen Wechselstrom spielt eine Richtung der Polung einer elektrischen Verbindung keine Rolle.

WO 2019/147774 A1 beschreibt eine Familie von Steckverbindern mit einer Z-Konfiguration des Ports innerhalb einer Buchse und eines Steckers. Die parallelen oberen und unteren Abschnitte der z-Konfiguration des Steckers enthalten Balkenkontakte, wobei der erste Balkenkontakt des Steckers gegenüber dem zweiten Balkenkontakt des Steckers um 180 Grad gedreht ist.

Für ein System aus verschiedenen elektrischen Steckverbindungen ist es bei sicherheitsrelevanten Anwendungen oft wünschenswert, dass eine Verwechslung von mehreren Steckelementen oder Aufnahmeelementen untereinander vermieden wird. Hierfür ist bekannt, jeder elektrischen Steckverbindung innerhalb eines solchen Systems eine charakteristische Form für Steckelement und Aufnahmeelement zuzuordnen, die diese elektrische Steckverbindung von den anderen elektrischen Steckverbindungen innerhalb des Systems unterscheidet.

Insbesondere für die Nutzung innerhalb einer Medizingeräteanordnung kann es besonders wichtig sein, Steckelemente verschiedener elektrischer Steckverbindungen nicht miteinander zu vertauschen, um nicht die Patientensicherheit eines zu versorgenden Patienten zu gefährden.

Aufgabe der vorliegenden Erfindung ist es, eine elektrische Steckverbindung für ein elektrisches Steckverbindungssystem bereitzustellen, die eine Umpolung durch ein Drehen des Steckelements erlaubt und dabei dennoch besonders vorteilhaft ein Vertauschen von Steckelementen verhindert.

Erfindungsgemäß wird zur Lösung dieser Aufgabe ein elektrisches Steckverbindungssystem gemäss Anspruch 1 vorgeschlagen.

Dabei bildet das Steckelement einen männlichen Teil der elektrischen Steckverbindung mit einem Steckbereich und das Aufnahmeelement einen weiblichen Teil der elektrischen Steckverbindung mit einer dem Steckbereich entsprechenden Aufnahme. Weiterhin sind das Steckelement und das Aufnahmeelement ausgebildet, im zusammengesteckten Zustand über mindestens zwei Kontaktbereiche eine elektrische Verbindung zwischen einem an dem Steckelement angeschlossenen Stecker-seitigen Kabel und einem an dem Aufnahmeelement angeschlossenen Aufnahme-seitigen Kabel bereitzustellen. Weiterhin ist der Steckbereich bezüglich einer gemäß einer Steckrichtung orientierten Steckachse der elektrischen Steckverbindung um 180° drehsymmetrisch ausgebildet, wodurch eine Umpolung der elektrischen Steckverbindung erlaubt ist. Allerdings ist der Steckbereich erfindungsgemäß nicht bezüglich einer beliebigen die Steckachse aufweisenden Bezugsebene spiegelsymmetrisch.

Im Rahmen der Erfindung wurde erkannt, dass ein Verzicht auf eine spiegelsymmetrische Ausbildung des Steckbereiches des Steckelements die Wahrscheinlichkeit eines versehentlichen Einsteckens eines fremden Steckelements reduziert, da viele handelsübliche Steckelemente spiegelsymmetrisch ausgebildet sind. Weiterhin wurde erkannt, dass eine Umpolung der elektrischen Steckverbindung bereits durch eine Drehsymmetrie um 180°, also insbesondere eine Punktsymmetrie, ermöglicht wird, ohne dass hierfür eine Spiegelsymmetrie des Steckbereich erforderlich ist.

Vorteilhaft wird durch die erfindungsgemäße elektrische Steckverbindung eine Umpolung erlaubt, so dass ein Nutzer besonders schnell und einfach die beiden Elemente zusammenstecken kann, ohne dafür die Orientierung des Steckbereichs zu überprüfen.

Aufgrund der einfachen Struktur kann die erfindungsgemäße elektrische Steckverbindung durch einfache, beispielsweise im Rahmen eines Spritzgussverfahrens hergestellte Teile bereitgestellt werden. Somit ist kein erhöhter Aufwand im Vergleich zur Herstellung bekannter handelsüblicher elektrischer Steckverbindungen notwendig.

Durch die fehlende Spiegelsymmetrie des Steckbereichs hat dieser erfindungsgemäße Steckbereich eine charakteristische Form, durch die eine Fehlbedienung durch einen Nutzer der elektrischen Steckverbindung vermieden wird. Insbesondere können handelsübliche spiegelsymmetrisch ausgebildete Steckelemente nicht oder zumindest teilweise nicht in Kombination mit dem erfindungsgemäßen Aufnahmeelement verwendet werden.

Die charakteristische Form des erfindungsgemäßen Steckbereiches erhöht vorteilhaft die Sicherheit, da die beiden durch die elektrische Steckverbindung zusammengesetzten elektrischen Komponenten vom selben Hersteller oder von miteinander kooperierenden Herstellern ausgeliefert wurden. Daher ist eine Kompatibilität beider Komponenten wahrscheinlich. Hierdurch wird eine besonders sichere Bedienung des entsprechenden elektrischen Systems gewährleistet, was insbesondere im medizinischen Umfeld vorteilhaft zu einer erhöhten Patientensicherheit führen kann.

Die mindestens zwei Kontaktbereiche werden vorzugsweise über mindestens zwei Kontaktpins an einem der beiden Elemente der elektrischen Steckverbindung und entsprechende Pin-Aufnahmen an dem jeweils anderen Element bereitgestellt. Die beiden Kontaktbereiche werden dabei über ein leitendes Material, insbesondere über ein Metall bereitgestellt. Das Vorsehen geeigneter Materialien und/oder geeigneter Legierungen für die Realisierung von Kontaktbereichen innerhalb einer elektrischen Steckverbindung ist dem Fachmann bekannt, so dass auf Details zu der Materialauswahl und der Herstellung im Folgenden nicht eingegangen wird.

Die Steckrichtung ist diejenige Richtung, in die das Steckelement bewegt werden muss, um den Steckbereich in die Aufnahme des Aufnahmeelements einzubringen. Die Steckachse ist dabei eine in Steckrichtung orientierte Achse, die durch den Steckbereich verläuft und dabei diejenige Symmetrieachse bildet, um die der Steckbereich um 180° drehsymmetrisch ausgebildet ist. Vorzugsweise ist der erfindungsgemäße Steckbereich entsprechend punktsymmetrisch um denjenigen Punkt, an dem die Steckachse den Steckbereich schneidet.

Die Befestigung von Stecker-seitigem und Aufnahme-seitigem Kabel an den beiden Elementen erfolgt vorzugsweise in einer dem Fachmann für elektrische Steckverbindungen bekannten Art und Weise, so dass darauf im Folgenden nicht detailliert eingegangen wird.

Die Bezugsebene ist eine beliebige Ebene derjenigen Ebenen, die die Steckachse aufweisen. Erfindungsgemäß gibt es also keine Ebene, zu der der Steckbereich spiegelsymmetrisch ausgebildet ist.

Nachfolgend werden bevorzugte Ausführungsformen der erfindungsgemäßen Steckverbindung beschrieben.

Erfindungsgemäß ist der Steckbereich im Wesentlichen wellenförmig, z-förmig oder s-förmig ausgebildet. Diese Formen erfüllen in besonders einfacher Weise die erfindungsgemäße Bedingung, dass der Steckbereich punktsymmetrisch ausgebildet ist, ohne dabei spiegelsymmetrisch zu sein. Besonders vorteilhaft weisen insbesondere die z-Form und die s-Form jeweils an den äußeren Enden des jeweiligen Steckbereiches einen Bereich auf, der ein versehentliches Drehen, insbesondere ein Herausdrehen, des Steckelements im zusammengesetzten Zustand der elektrischen Steckverbindung verhindert. Diese erhöhte Verdreh-Sicherheit sorgt für eine besonders zuverlässige Verbindung der beiden Elemente der erfindungsgemäßen Steckverbindung. Z-Form und S-Form bilden hierbei ein besonders eckiges und ein besonders geschwungenes Beispiel für die gleiche Grundform. Unter wellenförmig kann daher im Sinne der Erfindung auch Zacken-förmig oder Zahn-förmig oder Ähnliches verstanden werden.

In einer bevorzugten Ausführungsform weist das Aufnahmeelement mindestens zwei Kontaktpins auf, die jeweils zu dem jeweiligen Kontaktbereich der mindestens zwei Kontaktbereiche gehören. In dieser Ausführungsform sind dabei mindestens zwei der mindestens zwei Kontaktpins außerhalb der Steckachse angeordnet. Über diese außerhalb der Steckachse befindlichen Kontaktpins wird sichergestellt, dass eine Umpolung der elektrischen Steckverbindung durch ein Drehen des Steckelements und mithin ein entsprechendes Drehen der über die Kontaktpins bereitgestellten Kontaktbereiche möglich ist. In einer Variante dieser Ausführungsform weist die elektrische Steckverbindung zusätzlich einen Kontaktbereich auf, der im Bereich der Steckachse angeordnet ist, beispielsweise über einen weiteren Kontaktpin, insbesondere über einen dritten Kontaktpin, im Bereich der Steckachse. Verschiedene Ausgestaltungen von Kontaktpins sind bekannt und werden daher im Folgenden nicht detailliert erläutert.

In einer weiteren Ausführungsform weisen das Steckelement und das Aufnahmeelement einen jeweiligen Griffbereich mit einer jeweiligen Griffstruktur auf. Vorteilhaft ist eine solche Griffstruktur mit einer strukturierten Oberfläche, insbesondere mit einer ergonomisch geformten Oberfläche ausgebildet. Dadurch erlaubt die jeweilige Griffstruktur auch bei schlechter Sicht eine derartige Orientierung einer Winkellage von Steckelement und Aufnahmeelement zueinander, dass ein Zusammenstecken aufgrund dieser aufeinander abgestimmten Griffstrukturen einfach möglich ist, insbesondere ohne ein genaues Studium der Struktur des Steckbereich möglich ist, insbesondere intuitiv möglich ist.

Erfindungsgemäß wird zum Lösen der oben genannten Aufgabe ein elektrisches Steckverbindungssystem mit mindestens zwei elektrischen Steckverbindungen gemäß mindestens einer der vorhergehenden Ausführungsformen vorgeschlagen.

Dabei weist eine erste elektrische Steckverbindung der mindestens zwei elektrischen Steckverbindungen verglichen mit einer zweiten elektrischen Steckverbindung der mindestens zwei elektrischen Steckverbindungen einen ersten Steckbereich auf, der spiegelverkehrt zu einem zweiten Steckbereich der zweiten elektrischen Steckverbindung ist.

Das erfindungsgemäße elektrische Steckverbindungssystem weist die Vorteile der elektrischen Steckverbindung auf und zusätzlich den Vorteil, dass die beiden Steckelemente im Falle einer Verwechslung durch einen Anwender nicht in die Aufnahme des jeweils nicht zugehörigen Aufnahmeelements eingesteckt werden können. Weiterhin sind die beiden Steckelemente des elektrischen Steckverbindungssystems aufgrund ihrer strukturellen Ähnlichkeit unmittelbar als zu demselben Steckverbindungssystem zugehörig erkennbar. So ist die Struktur der beiden Steckbereiche identisch und lediglich eine gespiegelte Formgebung, die ein versehentliches Umstecken des Steckelements in das jeweils andere Aufnahmeelement verhindert, unterscheidet die beiden Steckbereiche voneinander.

In einer besonders bevorzugten Ausführungsform bilden das jeweilige Steckelement mit dem jeweiligen Stecker-seitigen Kabel eine Mehrwegkomponente und das jeweilige Aufnahmeelement mit dem jeweiligen Aufnahme-seitigen Kabel eine Einwegkomponente des elektrischen Steckverbindungssystems. Diese Ausführungsform ist insbesondere in einem sterilen Umfeld vorteilhaft, da das Steckelement typischerweise einfacher zu reinigen ist als das Aufnahmeelement. Wenn eine der beiden Komponenten wiederverwendet werden sollte, stellt daher das Steckelement die einfacher zu reinigende Variante dar. Das Vorsehen einer Einwegkomponente kann neben hygienischen Aspekten auch aufgrund einer geringen Lebensdauer von entsprechenden elektronischen Komponenten oder aufgrund einer geringen Akzeptanz von entsprechenden gebrauchten Komponenten durch den Anwender vorteilhaft sein.

In einer weiteren Ausführungsform sind die Stecker-seitigen Kabel ausgebildet, das elektrische Steckverbindungssystem jeweils mit einer Stromversorgungseinheit zu verbinden. Vorteilhaft sind weiterhin die Aufnahme-seitigen Kabel ausgebildet, das elektrische Steckverbindungssystem jeweils mit einem mit Strom zu versorgenden Gerät zu verbinden. In einer bevorzugten Variante dieser Ausführungsform ist das Aufnahme-seitige Kabel zusammen mit dem zu versorgenden Gerät als Einwegkomponente ausgebildet, wohingegen die Stromversorgungseinheit mit dem Stecker-seitigen Kabel eine Mehrwegkomponente ist.

In einer vorteilhaften Ausführungsform des elektrischen Steckverbindungssystems sind Steckelement und Aufnahmeelement der ersten elektrischen Steckverbindung zumindest teilweise in einer ersten Farbe ausgebildet und Steckelement und Aufnahmeelement der zweiten elektrischen Steckverbindung zumindest teilweise in einer zweiten Farbe ausgebildet. Dabei sind in dieser Ausführungsform die erste und die zweite Farbe unterschiedlich voneinander. In dieser Ausführungsform kann vorteilhaft aufgrund der Farben ohne weiteres auf eine jeweilige Zugehörigkeit von Aufnahmeelement und Steckelement zu einer gemeinsamen elektrischen Steckverbindung geschlossen werden. Insbesondere müssen nicht die aufgrund der symmetrischen Ausgestaltung ähnlichen Steckbereiche miteinander verglichen werden.

In einer weiteren Ausführungsform weisen ein jeweiliges Steckelement und das zugehörige Aufnahmeelement einen jeweiligen Griffbereich mit einer jeweiligen Griffstruktur auf. Vorteilhaft ist diese Griffstruktur charakteristisch ausgebildet. Dadurch erlaubt die jeweilige Griffstruktur auch bei schlechter Sicht eine derartige Orientierung einer Winkellage von Steckelement und Aufnahmeelement zueinander, dass ein Zusammenstecken aufgrund dieser aufeinander abgestimmten Griffstrukturen einfach möglich ist, insbesondere ohne ein genaues Studium der Struktur des Steckbereich möglich ist, insbesondere intuitiv möglich ist. In einer besonders bevorzugten Variante unterscheiden sich die beiden Griffstrukturen der beiden elektrischen Steckverbindungen derart deutlich voneinander, dass aufgrund der Griffstrukturen intuitiv gar nicht erst eine Kontaktierung eines Steckelements mit dem nicht dazugehörigen Aufnahmeelement durch einen Anwender versucht wird.

In einer weiteren Ausführungsform sind die beiden Steckbereiche des elektrischen Steckverbindungssystems derart ausgebildet, dass ein spiegelsymmetrisch ausgebildeter Prüfstecker in beide Aufnahmeelemente der mindestens zwei elektrischen Steckverbindungen eingebracht werden kann und eine elektrische Verbindung zwischen Prüfstecker und Aufnahmeelement erlaubt. Hierbei hat der Steckbereich des Prüfsteckers eine kleinere Fläche als der Steckbereich des erfindungsgemäßen Steckelements. Hierdurch wird eine Wartung des elektrischen Steckverbindungsystems vereinfacht, da durch einen universell einsetzbaren Prüfstecker eine Prüfung der Funktionsfähigkeit des jeweiligen Aufnahmeelements und der jeweiligen daran angeschlossenen Geräte möglich ist.

Gemäß einem weiteren Aspekt der Erfindung wird zur Lösung der oben genannten Aufgabe eine Medizingeräteanordnung mit einem elektrischen Steckverbindungssystem gemäß einer der vorhergehenden Ausführungsformen vorgeschlagen.

Die vorhergenannten Vorteile der elektrischen Steckverbindung und des elektrischen Steckverbindungssystems sind besonders vorteilhaft ausgeprägt für die Medizingeräteanordnung gemäß dem weiteren Aspekt der Erfindung. So unterstützt eine sichere Zuordnung zwischen Steckelement und Aufnahmeelement eine sichere und zuverlässige Bedienung der Medizingeräteanordnung und somit die Patientensicherheit.

Weiterhin vorteilhaft ist durch die erfindungsgemäß zulässige Umpolung eine schnelle Bedienung der elektrischen Steckverbindung auch unter schlechten Lichtverhältnissen möglich, was eine besonders schnelle Bedienung der erfindungsgemäßen Medizingeräteanordnung erlaubt.

Schließlich erlaubt die charakteristische Form des jeweiligen erfindungsgemäßen Steckbereichs eine sichere Zuordnung von Komponenten eines Herstellers der Medizingeräteanordnung. Hierdurch werden Probleme mit der Kompatibilität von Komponenten verschiedener Hersteller vermieden. Somit wird eine sichere Nutzung der Medizingeräteanordnung unterstützt und somit die Patientensicherheit verbessert.

In einer bevorzugten Ausführungsform der erfindungsgemäßen Medizingeräteanordnung ist eine jeweilige Steckverbindung einem jeweiligen beheizbaren Schlauch eines Schlauchsystems der Medizingeräteanordnung zugeordnet. Dabei wird über die jeweilige Steckverbindung eine Heizung, insbesondere eine Heizung eines Atemgasanfeuchters, des zugeordneten beheizbaren Schlauchs mit Strom versorgt. Da eine derartige Heizung typischerweise über einen Widerstandsdraht realisiert ist, ist eine Umpolung der elektrischen Steckverbindung ohne eine negative Auswirkung auf die Funktionsfähigkeit der entsprechenden Medizingeräteanordnung möglich. Das Verhindern eines Verwechselns der beiden Steckelemente der beiden Steckverbindungen verhindert in dieser Ausführungsform in einer bevorzugten Variante, dass inspiratorischer und exspiratorischer Heizanschluss für den entsprechenden inspiratorischen und exspiratorischen Beatmungsschlauch miteinander vertauscht werden. Da der inspiratorische Heizanschluss elektrisch geregelt ist und der exspiratorische Heizanschluss nicht elektrisch geregelt ist, ist eine zuverlässige Verhinderung einer solchen Vertauschung besonders vorteilhaft für das Sicherstellen der Funktionsfähigkeit der Medizingeräteanordnung.

In einer besonders vorteilhaften Variante der vorhergehenden Ausführungsform ist das jeweilige Aufnahmeelement über das jeweilige Aufnahme-seitige Kabel mit dem zugeordneten beheizbaren Schlauch nicht-lösbar verbunden. Vorzugsweise bilden dabei Aufnahmeelement, Aufnahme-seitiges Kabel und beheizbarer Schlauch zusammen eine Einwegkomponente der Medizingeräteanordnung. In dieser Variante wird vorteilhaft ausgenutzt, dass es sich bei einem Beatmungsschlauch typischerweise aufgrund hygienischer Aspekte um eine Einwegkomponente bei medizinischen Anwendungen handelt. Da das Steckelement besonders einfach zu reinigen ist, ist es daher vorteilhaft, lediglich die Aufnahme-seitigen Komponenten als Einwegkomponenten vorzusehen und die Steck-seitigen Komponenten derart bereitzustellen, dass eine einfache Reinigung und/oder Desinfektion möglich ist.

Die Erfindung soll nun anhand von in den Figuren schematisch dargestellten, vorteilhaften Ausführungsbeispielen näher erläutert werden. Von diesen zeigen im Einzelnen:
- Fig. 1: eine schematische Darstellung eines ersten Ausführungsbeispiels einer elektrischen Steckverbindung für ein erfindungsgemäßes Steckverbindungssystem;
- Fig. 2, 3, 4: eine jeweilige schematische Darstellung von drei verschiedenen Ausführungsbeispielen eines Steckbereichs der elektrischen Steckverbindung, wobei ein wellenförmige Steckbereich (Fig. 2), ein z-förmiger Steckbereich (Fig. 3) und ein s-förmiger Steckbereich (Fig. 4) gezeigt sind;
- Fig. 5: eine schematische Darstellung eines ersten Ausführungsbeispiels eines erfindungsgemäßen elektrischen Steckverbindungssystems;
- Fig. 6: eine schematische Darstellung eines zweiten Ausführungsbeispiels des erfindungsgemäßen elektrischen Steckverbindungssystems;
- Fig. 7: eine schematische Darstellung eines Ausführungsbeispiels einer Medizingeräteanordnung gemäß einem weiteren Aspekt der Erfindung.

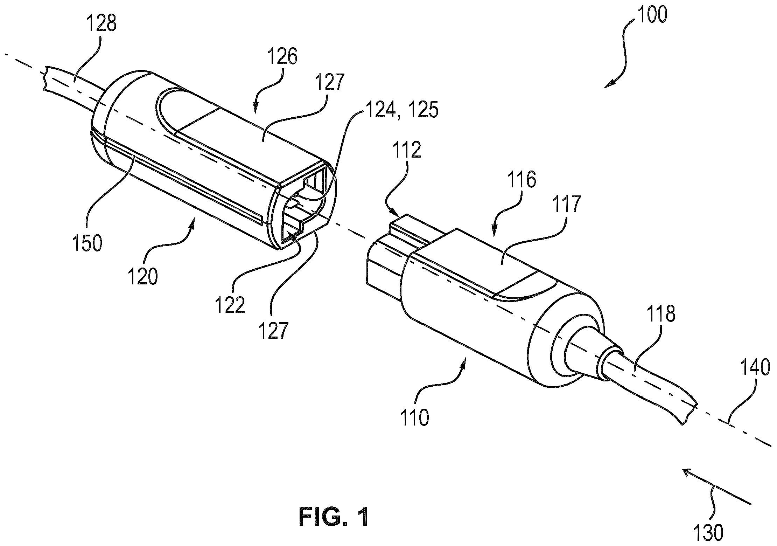

Fig. 1 zeigt eine schematische Darstellung eines ersten Ausführungsbeispiels einer elektrischen Steckverbindung 100 für ein erfindungsgemäßes Steckverbindungssystem.

Die elektrische Steckverbindung 100 ist zur formschlüssigen Verbindung eines Steckelements 110 mit einem Aufnahmeelement 120 innerhalb einer Medizingeräteanordnung ausgebildet.

Das Steckelement 110 bildet einen männlichen Teil der elektrischen Steckverbindung 100 mit einem Steckbereich 112, der ein Kontaktelement (nicht in Fig. 1 dargestellt) zum Bereitstellen einer elektrischen Verbindung mit einem entsprechenden Kontaktgegenelement 124 des Aufnahmeelements 120 aufweist. Das Steckelement 110 weist weiterhin einen Stecker-seitigen Griffbereich 116 auf, der zwei gegenüberliegende abgeflachte Bereiche 117 umfasst. An dem Steckelement 110 ist ein Stecker-seitiges Kabel 118 angeschlossenen.

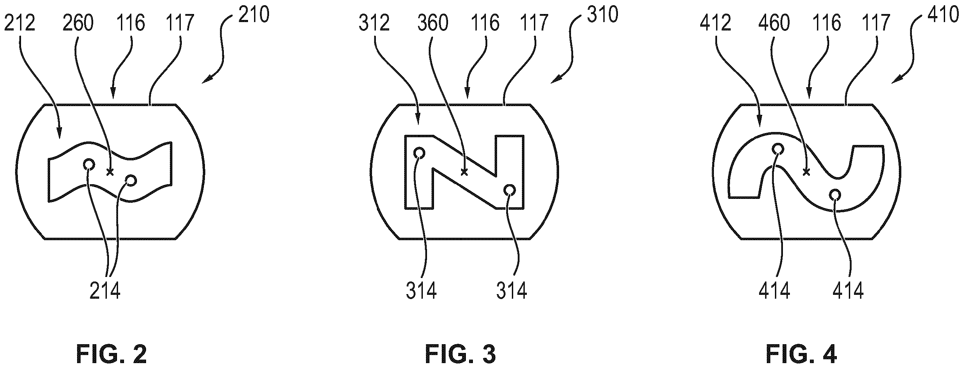

Das Aufnahmeelement 120 bildet einen weiblichen Teil der elektrischen Steckverbindung 100 mit einer dem Steckbereich 112 entsprechenden Aufnahme 122. Das Aufnahmeelement 120 umfasst das Kontaktgegenelement 124, welches vorliegend als ein Kontaktpin 125 ausgebildet ist, während das Kontaktelement des Steckelements 110 eine Kontaktaufnahme aufweist. Im zusammengesteckten Zustand bilden Kontaktpin und Kontaktaufnahme mindestens zwei Kontaktbereiche, über die eine elektrische Verbindung zwischen dem Stecker-seitigen Kabel 118 und einem an dem Aufnahmeelement 120 angeschlossenen Aufnahme-seitigen Kabel 128 bereitgestellt wird. Vorzugsweise werden die mindestens zwei Kontaktbereiche durch mindestens zwei Kontaktpins bereitgestellt, wie in den Figuren 2 bis 4 gezeigt. Das Aufnahmeelement 120 weist weiterhin einen Aufnahme-seitigen Griffbereich 126 auf, der ebenfalls zwei gegenüberliegende abgeflachte Bereiche 127 umfasst. Die beiden Griffbereiche 116, 126 sind derart aufeinander abgestimmt, dass intuitiv eine geeignete Winkellage von Steckelement 110 und Aufnahmeelement 120 zueinander dadurch gefunden wird, dass die jeweiligen abgeflachten Bereiche 117, 127 zusammengebracht werden.

Durch die Richtung, in die das Steckelement 110 gebracht werden muss, um in das Aufnahmeelement 120 hineingesteckt zu werden, wird die Steckrichtung 130 vorgegeben.

Der Steckbereich 112 ist bezüglich einer gemäß der Steckrichtung 130 orientierten Steckachse 140 der elektrischen Steckverbindung 100 um 180° drehsymmetrisch ausgebildet. Die drehsymmetrische Struktur des Steckbereichs 112 wird in dem dargestellten Ausführungsbeispiel durch den im Wesentlichen z-förmigen Steckbereich 112 realisiert. Andere Formen des Steckbereichs sind erfindungsgemäß ebenfalls möglich, wie in den Figuren 2 bis 4 dargestellt. Durch die Drehsymmetrie ist eine Umpolung der elektrischen Steckverbindung erlaubt, wodurch ein schnelles Zusammenstecken der erfindungsgemäßen elektrischen Steckverbindung 100 möglich ist. Erfindungsgemäß sind der Steckbereich 112 und somit auch die entsprechende Aufnahme 122 jedoch nicht bezüglich einer beliebigen die Steckachse 140 aufweisenden Bezugsebene spiegelsymmetrisch. Durch diese charakteristische Struktur des Steckbereichs 112 kann ein versehentliches Zusammenstecken von nicht zusammengehörigen Paaren aus Stecker und Aufnahme vermieden werden. Insbesondere kann ein versehentliches Zusammenstecken mit bekannten, handelsüblichen Steckern, die typischerweise zum Erlauben einer Umpolung spiegelsymmetrisch ausgebildet sind, vermieden werden.

In dem dargestellten Ausführungsbeispiel ist besonders vorteilhaft, dass ein Zusammenführen der Winkellage der abgeflachten Bereiche 117, 127 des Steckelements 110 und des Aufnahmeelements 120 stets aufgrund der 180°-Drehsymmetrie des Steckbereichs 112 zu einer Konstellation führen, in der der Steckbereich 112 in die Aufnahme 122 eingeführt werden kann.

Weiterhin ist in Fig. 1 gezeigt, dass die Ummantelung des Aufnahmeelements 120 der elektrischen Steckverbindung 100 in dem dargestellten Ausführungsbeispiel über zwei Halbschalen aus Kunststoff bereitgestellt ist. Vorzugsweise werden die zwei Halbschalen über ein Spritzgussverfahren oder dergleichen hergestellt. Die beiden Halbschalen können an einem entsprechenden Verbindungsbereich 150 miteinander verbunden werden. In dem dargestellten Ausführungsbeispiel erfolgt dies über eine formschlüssige Verbindung. Weiterhin erfolgt die Ummantelung mit den zwei Halbschalen in dem dargestellten Ausführungsbeispiel nur für das Aufnahmeelement 120, wohingegen das Steckelement 110 durch Umspritzen des Stecker-seitigen Kabels 118 in einem Formwerkzeug hergestellt wurde. Hierdurch kann das Aufnahmeelement 110 besonders günstig hergestellt werden, was insbesondere vorteilhaft ist falls das Aufnahmeelement 110 eine Einwegkomponente ist. In einem nicht dargestellten Ausführungsbeispiel ist die Ummantelung von Aufnahmeelement und Steckelement über zwei Halbschalen ausgebildet. In einem weiteren nicht dargestellten Ausführungsbeispiel sind beide Ummantelungen durch ein Umspritzen hergestellt.

Die genaue Struktur einer solchen Ummantelung sowie eine geeignete Befestigung des jeweiligen Kabels mit dieser Ummantelung sind auf dem Gebiet der Steckverbindungen bekannt und werden daher im Folgenden nicht detailliert erläutert.

Die Figuren 2 bis 4 zeigen eine jeweilige schematische Darstellung von drei verschiedenen Ausführungsbeispielen des Steckbereichs 212, 312, 412 der elektrischen Steckverbindung 100. Dabei werden ein wellenförmiger Steckbereich 212 (Fig. 2), ein z-förmiger Steckbereich 312 (Fig. 3) und ein s-förmiger Steckbereich 412 (Fig. 4) gezeigt.

Die dargestellten drei Strukturen für den Steckbereich 212, 312, 412 sind lediglich beispielhaft für eine Vielzahl möglicher Strukturen, die im Sinne der Erfindung um 180° drehsymmetrisch, also auch punktsymmetrisch, ausgebildet sind, ohne dabei bezüglich einer beliebigen entsprechend der Steckachse orientierten Bezugsebene spiegelsymmetrisch zu sein.

In den Figuren 2 bis 4 wird durch ein Kreuz 260, 360, 460 derjenige Schnittpunkt gekennzeichnet, an dem die Steckachse den entsprechenden Steckbereich 212, 312, 412 schneidet.

Weiterhin ist in den drei in den Figuren 2 bis 4 dargestellten Ausführungsbeispielen die Ummantelung des jeweiligen Steckelements 210, 310, 410 ähnlich ausgebildet mit zwei gegenüberliegenden Bereichen 117, die den jeweiligen Griffbereich 116 bilden. Schließlich weist jeder der drei Steckbereiche 212, 312, 412 zwei Kontaktelemente 214, 314, 414 auf, die zeigen, dass die entsprechenden nicht dargestellten gegenüberliegenden Kontaktpins des entsprechenden Aufnahmeelements außerhalb der Steckachse angeordnet sind.

In einem nicht dargestellten Ausführungsbeispiel weist die erfindungsgemäße elektrische Steckverbindung mehr als zwei Kontaktgegenelemente und/oder mehr als zwei Kontaktelemente, insbesondere Kontaktpins auf.

In einem weiteren nicht dargestellten Ausführungsbeispiel weist der Griffbereich des Steckelements und/oder des Aufnahmeelements eine besonders ergonomische Struktur auf, die verschieden von dem dargestellten abgeflachten Bereich 117 ist.

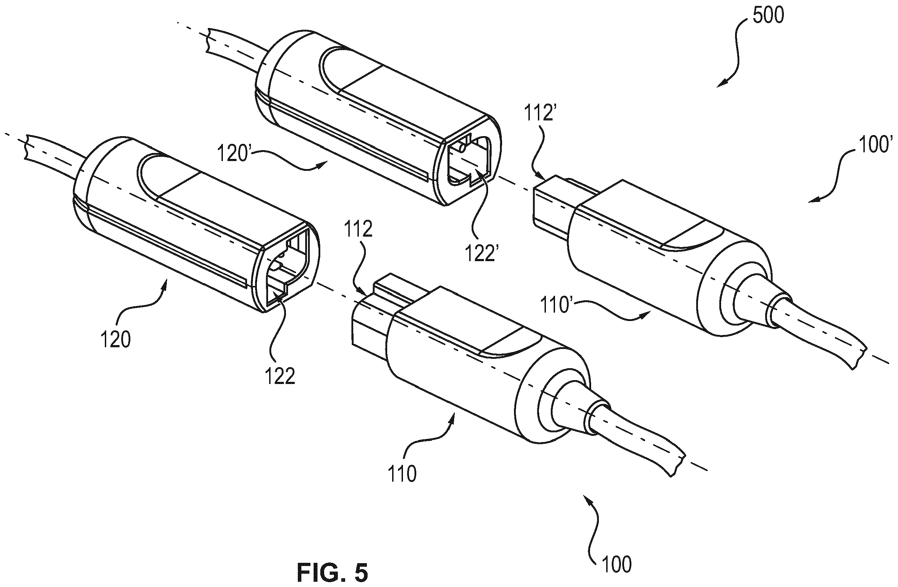

Fig. 5 zeigt eine schematische Darstellung eines ersten Ausführungsbeispiels eines erfindungsgemäßen elektrischen Steckverbindungssystems 500.

Das elektrische Steckverbindungssystem 500 umfasst mindestens zwei elektrische Steckverbindungen 100, 100`. Die elektrische Steckverbindung 100' unterscheidet sich nur dadurch von der elektrischen Steckverbindung 100, dass der Steckbereich 112' und die entsprechende Aufnahme 122` im Vergleich zur dem Steckbereich 112 und der entsprechenden Aufnahme 122 gespiegelt, also spiegelverkehrt, sind. Aufgrund der nicht spiegelsymmetrisch Struktur von Steckbereich 112, 112` und Aufnahme 122, 122' kann daher nicht die zweite Aufnahme 122` mit dem ersten Steckbereich 112 kombiniert werden, und umgekehrt kann auch nicht die erste Aufnahme 122 mit dem zweiten Steckbereich 112` kombiniert werden.

Ansonsten weisen die beiden elektrischen Steckverbindungen 100, 100' den gleichen Aufbau und somit die gleichen strukturellen Merkmale auf.

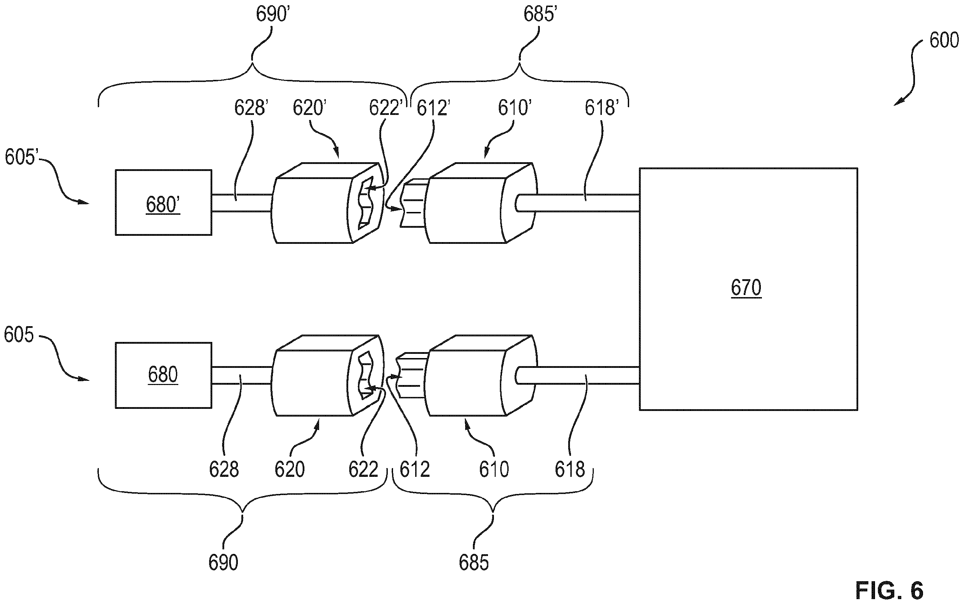

Fig. 6 zeigt eine schematische Darstellung eines zweiten Ausführungsbeispiels des erfindungsgemäßen elektrischen Steckverbindungssystems 600.

Das elektrische Steckverbindungssystem 600 unterscheidet sich dadurch von dem in Fig. 5 gezeigten Steckverbindungssystem 500, dass die beiden Steckbereiche 612, 612' und die beiden entsprechenden Aufnahmen 622, 622` nicht z-förmig sind wie bei dem Steckverbindungssystem 500, sondern wellenförmig. Erfindungsgemäß handelt es sich bei den beiden Wellenformen der dargestellten Aufnahmen 622, 622' um zueinander gespiegelte Wellenformen. Dadurch können Aufnahmen und Steckbereiche nicht miteinander vertauscht werden.

Weiterhin unterscheidet sich das elektrische Steckverbindungssystem 600 von dem Steckverbindungssystem 500 dadurch, dass die Stecker-seitigen Kabel 618, 618` ausgebildet sind, das elektrische Steckverbindungssystem 600 jeweils mit einer Stromversorgungseinheit 670 zu verbinden, und die Aufnahme-seitigen Kabel 628, 628` ausgebildet sind, das elektrische Steckverbindungssystem 600 jeweils mit einem mit Strom zu versorgenden Gerät 680, 680' zu verbinden. In dem dargestellten Ausführungsbeispiel handelt es sich bei den beiden mit Strom zu versorgenden Geräten 680, 680' um medizinische Geräte, insbesondere um ein jeweiliges Heizelement eines medizinischen Gerätes.

Schließlich handelt es sich bei den beiden Steckelementen 610, 610` zusammen mit den Stecker-seitigen Kabeln (618, 618') um Mehrwegkomponenten (685, 685'), während die beiden Aufnahmeelemente 620, 620' Einwegkomponenten sind. In dem dargestellten Ausführungsbeispiel sind die beiden Aufnahmeelemente 620, 620` zusammen mit den beiden Aufnahme-seitigen Kabeln 628, 628` und den beiden medizinischen Geräten 680, 680' kombinierte Einwegkomponenten 690, 690`.

Nicht dargestellt in Fig. 6 ist die farbliche Gestaltung des elektrischen Steckverbindungssystems 600. So sind das Steckelement 610 und das Aufnahmeelement 620 der ersten elektrischen Steckverbindung 605 in einer ersten Farbe bereitgestellt und das Steckelement 610` sowie das Aufnahmeelement 620` der zweiten elektrischen Steckverbindung 605' in einer zweiten Farbe bereitgestellt, wobei sich die beiden Farben unterscheiden.

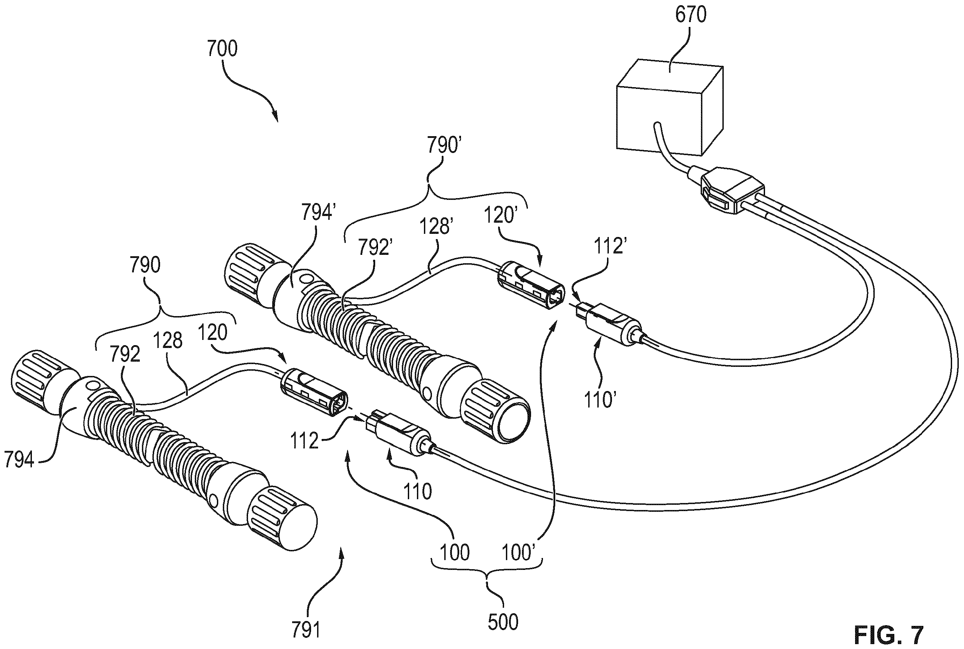

Fig. 7 zeigt eine schematische Darstellung eines Ausführungsbeispiels einer Medizingeräteanordnung 700 gemäß einem weiteren Aspekt der Erfindung.

Die erfindungsgemäße Medizingeräteanordnung 700 umfasst das erfindungsgemäße elektrische Steckverbindungssystem 500.

Zusätzlich zu den erfindungsgemäßen Komponenten des elektrischen Steckverbindungssystems 500 umfasst die Medizingeräteanordnung 700 in dem dargestellten Ausführungsbeispiel ein beheizbares Schlauchsystem 791, das durch die Stromversorgungseinheit 670 über das elektrische Steckverbindungssystem 500 mit Strom zu versorgen ist. Dabei ist eine jeweilige Steckverbindung 110, 110' einem jeweiligen beheizbaren Schlauch 792, 792` des Schlauchsystems der Medizingeräteanordnung 700 zugeordnet.

Über die jeweilige Steckverbindung wird eine jeweilige Heizung 794, 794` des entsprechenden beheizbaren Schlauches 792, 792`, vorliegend insbesondere eine Heizung eines Atemgasanfeuchters, mit Strom versorgt.

In dem in Fig. 7 dargestellten Ausführungsbeispiel handelt es sich bei den beiden beheizbaren Schläuchen 792, 792` um einen inspiratorischen Beatmungsschlauch 792 und um einen exspiratorischen Beatmungsschlauch 792`. Da die inspiratorische Heizspannung vorliegend elektrisch geregelt ist und die exspiratorische Heizspannung nicht, muss ein Vertauschen der Steckelemente 110, 110' untereinander aus Gründen der Patientensicherheit vermieden werden. Durch die erfindungsgemäße charakteristische Struktur des jeweiligen Steckbereichs, bei der die beiden elektrischen Steckverbindungen 100, 100' zueinander gespiegelte Steckbereich 112, 112` aufweisen, kann ein Vertauschen der Steckelemente 110, 110' vorteilhaft nicht erfolgen.

Weiterhin ist in dem dargestellten Ausführungsbeispiel das jeweilige Aufnahmeelement 120, 120' über das jeweilige Aufnahme-seitige Kabel 128, 128` mit dem zugeordneten beheizbaren Schlauch 792, 792` nicht-lösbar verbunden. Hierbei bilden eine jeweilige Kombination aus Aufnahmeelement 120, 120', Aufnahme-seitigem Kabel 128, 128` und beheizbarem Schlauch 792, 792` zusammen eine kombinierte Einwegkomponente 790, 790` der Medizingeräteanordnung.

Die erfindungsgemäße elektrische Steckverbindung kann natürlich vorteilhaft in weiteren nicht dargestellten Ausführungsbeispielen für andere medizinische Geräte und/oder medizinische Komponenten verwendet werden. Insbesondere für eine Medizingeräteanordnung, die zwei mit Strom zu versorgende Elemente aufweist, deren elektrische Verbindungen nicht vertauscht werden dürfen, kann besonders vorteilhaft ein derartiges Vertauschen verhindert werden. In einer derartigen Anwendung wird durch die elektrische Steckverbindung und/oder das erfindungsgemäße elektrische Steckverbindungssystem die Patientensicherheit unterstützt und eine Handhabung der entsprechenden Steckverbindung durch den Anwender vereinfacht.

### Bezugszeichenliste

- 100, 100', 605, 605`: elektrische Steckverbindung
- 110, 110', 210, 310, 410, 610, 610': Steckelement
- 112, 112', 212, 312, 412, 612, 612': Steckbereich
- 116, 126: Griffbereich
- 117, 127: abgeflachter Bereich
- 118, 618, 618`: Stecker-seitiges Kabel
- 120, 120', 620, 620`: Aufnahmeelement
- 122, 122', 622, 622`: Aufnahme
- 124: Kontaktgegenelement
- 125: Kontaktpin
- 128, 128', 628, 628`: Aufnahme-seitiges Kabel
- 130: Steckrichtung
- 140: Steckachse
- 150: Verbindungsbereich
- 214, 314, 414: Kontaktelement
- 260, 360, 460: Schnittpunkt
- 500, 600: elektrisches Steckverbindungssystem
- 670: Stromversorgungseinheit
- 680, 680': zu versorgendes Gerät
- 685, 685`: Mehrwegkomponente
- 690, 690', 790, 790': kombinierte Einwegkomponente
- 700: Medizingeräteanordnung
- 791: Schlauchsystem
- 792, 792`: Schlauch
- 794, 794`: Heizung

## Patentansprüche

1. Elektrisches Steckverbindungssystem (500, 600), mit mindestens zwei elektrischen Steckverbindungen (100, 100', 605, 605'), wobei eine jeweilige elektrische Steckverbindung von den mindestens zwei elektrischen Steckverbindungen ausgebildet ist zur formschlüssigen Verbindung eines Steckelements (110, 110', 210, 310, 410, 610, 610') mit einem Aufnahmeelement (120, 120', 620, 620') innerhalb einer Medizingeräteanordnung (700),
wobei das Steckelement (110, 110', 210, 310, 410, 610, 610') einen männlichen Teil der jeweiligen elektrischen Steckverbindung (100, 100', 605, 605') mit einem Steckbereich (112, 112', 212, 312, 412, 612, 612') bildet und wobei das Aufnahmeelement (120, 120', 620, 620') einen weiblichen Teil der jeweiligen elektrischen Steckverbindung (100, 100', 605, 605') mit einer dem Steckbereich (112, 112', 212, 312, 412, 612, 612') entsprechenden Aufnahme (122, 122', 622, 622') bildet, wobei Steckelement (110, 110', 210, 310, 410, 610, 610') und Aufnahmeelement (120, 120', 620, 620') ausgebildet sind, im zusammengesteckten Zustand über mindestens zwei Kontaktbereiche eine elektrische Verbindung zwischen einem an dem Steckelement (110, 110', 210, 310, 410, 610, 610') angeschlossenen Stecker-seitigen Kabel (118, 618, 618') und einem an dem Aufnahmeelement (120, 120', 620, 620') angeschlossenen Aufnahme-seitigen Kabel (128, 128', 628, 628') bereitzustellen,
wobei der Steckbereich (112, 112', 212, 312, 412, 612, 612') bezüglich einer gemäß einer Steckrichtung (130) orientierten Steckachse (140) der jeweiligen elektrischen Steckverbindung (100, 100', 605, 605') um 180° drehsymmetrisch ausgebildet ist und
dadurch eine Umpolung der jeweiligen elektrischen Steckverbindung (100, 100', 605, 605') erlaubt, ohne dabei bezüglich einer beliebigen die Steckachse (140) aufweisenden Bezugsebene spiegelsymmetrisch zu sein, wobei der Steckbereich (112, 112', 212, 312, 412, 612, 612') im Wesentlichen wellenförmig, z-förmig oder s-förmig ausgebildet ist,
**dadurch gekennzeichnet,**
**dass** eine erste elektrische Steckverbindung (100, 100', 605, 605') der mindestens zwei elektrischen Steckverbindungen (100, 100', 605, 605') verglichen mit einer zweiten elektrischen Steckverbindung (100, 100', 605, 605') der mindestens zwei elektrischen Steckverbindungen (100, 100', 605, 605') einen ersten Steckbereich (112, 112', 212, 312, 412, 612, 612') aufweist, der spiegelverkehrt zu einem zweiten Steckbereich (112, 112', 212, 312, 412, 612, 612') der zweiten elektrischen Steckverbindung (100, 100', 605, 605') ist.

2. Elektrisches Steckverbindungssystem (500, 600) gemäß Anspruch 1, wobei die Stecker-seitigen Kabel (118, 618, 618') ausgebildet sind, das elektrische Steckverbindungssystem (500, 600) jeweils mit einer Stromversorgungseinheit (670) zu verbinden, und die Aufnahme-seitigen Kabel (128, 128', 628, 628') ausgebildet sind, das elektrische Steckverbindungssystem (500, 600) jeweils mit einem mit Strom zu versorgenden Gerät (680, 680') zu verbinden.

3. Elektrisches Steckverbindungssystem (500, 600) gemäß mindestens einem der Ansprüche 1 bis 2, wobei Steckelement (110, 110', 210, 310, 410, 610, 610') und Aufnahmeelement (120, 120', 620, 620') der ersten elektrischen Steckverbindung (100, 100', 605, 605') zumindest teilweise in einer ersten Farbe ausgebildet sind und Steckelement (110, 110', 210, 310, 410, 610, 610') und Aufnahmeelement (120, 120', 620, 620') der zweiten elektrischen Steckverbindung (100, 100', 605, 605') zumindest teilweise in einer zweiten Farbe ausgebildet sind, und wobei die erste und die zweite Farbe unterschiedlich voneinander sind.

4. Medizingeräteanordnung (700), mit einem elektrischen Steckverbindungssystem (500, 600) gemäß mindestens einem der Ansprüche 1 bis 3.

5. Medizingeräteanordnung (700) gemäß Anspruch 4, wobei eine jeweilige Steckverbindung (100, 100', 605, 605') einem jeweiligen beheizbaren Schlauch (792, 792') eines Schlauchsystems (791) der Medizingeräteanordnung (700) zugeordnet ist, und wobei über die jeweilige Steckverbindung (100, 100', 605, 605') eine Heizung (794, 794'), insbesondere eine Heizung eines Atemgasanfeuchters, des zugeordneten beheizbaren Schlauchs (792, 792') mit Strom versorgt wird.

6. Medizingeräteanordnung (700) gemäß Anspruch 5, wobei das jeweilige Aufnahmeelement (120, 120', 620, 620') über das jeweilige Aufnahme-seitige Kabel (128, 128', 628, 628') mit dem zugeordneten beheizbaren Schlauch (792, 792') nicht-lösbar verbunden ist, und wobei Aufnahmeelement (120, 120', 620, 620'), Aufnahme-seitiges Kabel (128, 128', 628, 628') und beheizbarer Schlauch (792, 792`) zusammen eine Einwegkomponente (690, 690', 790, 790') der Medizingeräteanordnung (700) bilden.

## Claims

1. Electrical plug-in connection system (500, 600) having at least two electrical plug-in connections (100, 100', 605, 605'), wherein a respective electrical plug-in connection is formed by the at least two electrical plug-in connections for positively connecting a plug-in element (110, 110', 210, 310, 410, 610, 610') to a receiving element (120, 120', 620, 620') within a medical device arrangement (700), wherein the plug-in element (110, 110', 210, 310, 410, 610, 610') forms a male part of the respective electrical plug-in connection (100, 100', 605, 605') with a plug-in region (112, 112', 212, 312, 412, 612, 612'), and wherein the receiving element (120, 120', 620, 620') forms a female part of the respective electrical plug-in connection (100, 100', 605, 605') with a receptacle (122, 122', 622, 622') corresponding to the plug-in region (112, 112', 212, 312, 412, 612, 612'), wherein the plug-in element (110, 110', 210, 310, 410, 610, 610') and the receiving element (120, 120', 620, 620') are designed to provide, in the plug-connected state via at least two contact regions, an electrical connection between a plug-side cable (118, 618, 618') connected to the plug-in element (110, 110', 210, 310, 410, 610, 610') and a receptacle-side cable (128, 128', 628, 628') connected to the receiving element (120, 120', 620, 620'), wherein the plug-in region (112, 112', 212, 312, 412, 612, 612') is designed to be rotationally symmetrical through 180° with respect to a plug-in axis (140), oriented in line with a plug-in direction (130), of the respective electrical plug-in connection (100, 100', 605, 605') and in this way allows reversal of polarity of the respective electrical plug-in connection (100, 100', 605, 605'), without being mirror-symmetrical with respect to an arbitrary reference plane having the plug-in axis (140), wherein the plug-in region (112, 112', 212, 312, 412, 612, 612') is of substantially wave-like, z-shaped or s-shaped design, **characterized in that** a first electrical plug-in connection (100, 100', 605, 605') of the at least two electrical plug-in connections (100, 100', 605, 605') compared with a second electrical plug-in connection (100, 100', 605, 605') of the at least two electrical plug-in connections (100, 100', 605, 605') has a first plug-in region (112, 112', 212, 312, 412, 612, 612') which is mirror-inverted with respect to a second plug-in region (112, 112', 212, 312, 412, 612, 612') of the second electrical plug-in connection (100, 100', 605, 605').

2. Electrical plug-in connection system (500, 600) according to Claim 1, wherein the plug-side cables (118, 618, 618') are designed to connect the electrical plug-in connection system (500, 600) to a power supply unit (670) in each case, and the receptacle-side cables (128, 128', 628, 628') are designed to connect the electrical plug-in connection system (500, 600) to a device (680, 680') to be supplied with power in each case.

3. Electrical plug-in connection system (500, 600) according to at least one of Claims 1 and 2, wherein the plug-in element (110, 110', 210, 310, 410, 610, 610') and the receiving element (120, 120', 620, 620') of the first electrical plug-in connection (100, 100', 605, 605') are at least partially formed in a first colour and the plug-in element (110, 110', 210, 310, 410, 610, 610') and the receiving element (120, 120', 620, 620') of the second electrical plug-in connection (100, 100', 605, 605') are at least partially formed in a second colour, and wherein the first and the second colour differ from one another.

4. Medical device arrangement (700) having an electrical plug-in connection system (500, 600) according to at least one of Claims 1 to 3.

5. Medical device arrangement (700) according to Claim 4, wherein a respective plug-in connection (100, 100', 605, 605') is associated with a respective heatable hose (792, 792') of a hose system (791) of the medical device arrangement (700), and wherein a heater (794, 794'), in particular a heater of a respiratory gas humidifier, of the associated heatable hose (792, 792') is supplied with power via the respective plug-in connection (100, 100', 605, 605').

6. Medical device arrangement (700) according to Claim 5, wherein the respective receiving element (120, 120', 620, 620') is non-releasably connected to the associated heatable hose (792, 792') via the respective receptacle-side cable (128, 128', 628, 628'), and wherein the receiving element (120, 120', 620, 620'), the receptacle-side cable (128, 128', 628, 628') and the heatable hose (792, 792') together form a single-use component (690, 690', 790, 790') of the medical device arrangement (700).

## Revendications

1. Système de connexion enfichable électrique (500, 600), comportant au moins deux connexions enfichables électriques (100, 100', 605, 605'),
dans lequel une connexion enfichable électrique respective parmi lesdites au moins deux connexions enfichables électriques est conçue pour la liaison par complémentarité de forme d'un élément enfichable (110, 110', 210, 310, 410, 610, 610') avec un élément de réception (120, 120', 620, 620') au sein d'un agencement d'appareils médicaux (700),
dans lequel l'élément enfichable (110, 110', 210, 310, 410, 610, 610') forme une partie mâle de la connexion enfichable électrique respective (100, 100', 605, 605') avec une zone d'enfichage (112, 112', 212, 312, 412, 612, 612') et l'élément de réception (120, 120', 620, 620') forme une partie femelle de la connexion enfichable électrique respective (100, 100', 605, 605') avec une zone de réception (122, 122', 622, 622') correspondant à la zone d'enfichage (112, 112', 212, 312, 412, 612, 612'),
dans lequel l'élément enfichable (110, 110', 210, 310, 410, 610, 610') et l'élément de réception (120, 120', 620, 620') sont conçus, à l'état enfiché, pour fournir, par l'intermédiaire d'au moins deux zones de contact, une liaison électrique entre un câble côté fiche (118, 618, 618') raccordé à l'élément enfichable (110, 110', 210, 310, 410, 610, 610') et un câble côté réception (128, 128', 628, 628') raccordé à l'élément de réception (120, 120', 620, 620'), dans lequel la zone d'enfichage (112, 112', 212, 312, 412, 612, 612') est conçue avec une symétrie de rotation de 180° par rapport à un axe d'enfichage (140), orienté selon une direction d'enfichage (130), de la connexion enfichable électrique respective (100, 100', 605, 605') et permet ainsi une inversion de polarité de la connexion enfichable électrique respective (100, 100', 605, 605') sans être symétrique par rapport à un plan de référence quelconque présentant l'axe d'enfichage (140),
dans lequel la zone d'enfichage (112, 112', 212, 312, 412, 612, 612') est conçue sensiblement en forme d'onde, en forme de Z ou en forme de S,
**caractérisé en ce qu'**une première connexion enfichable électrique (100, 100', 605, 605') desdites au moins deux connexions enfichables électriques (100, 100', 605, 605') présente, par rapport à une deuxième connexion enfichable électrique (100, 100', 605, 605') desdites au moins deux connexions enfichables électriques (100, 100', 605, 605'), une première zone d'enfichage (112, 112', 212, 312, 412, 612, 612') qui est inversée par rapport à une deuxième zone d'enfichage (112, 112', 212, 312, 412, 612, 612') de la deuxième connexion enfichable électrique (100, 100', 605, 605').

2. Système de connexion enfichable électrique (500, 600) selon la revendication 1, dans lequel les câbles côté fiche (118, 618, 618') sont respectivement conçus pour connecter le système de connexion enfichable électrique (500, 600) à une unité d'alimentation en courant (670), et les câbles côté réception (128, 128', 628, 628') sont respectivement conçus pour connecter le système de connexion enfichable électrique (500, 600) à un appareil (680, 680') à alimenter en courant.

3. Système de connexion enfichable électrique (500, 600) selon au moins l'une des revendications 1 à 2, dans lequel l'élément enfichable (110, 110', 210, 310, 410, 610, 610') et l'élément de réception (120, 120', 620, 620') de la première connexion enfichable électrique (100, 100', 605, 605') sont conçus au moins partiellement dans une première couleur et l'élément enfichable (110, 110', 210, 310, 410, 610, 610') et l'élément de réception (120, 120', 620, 620') de la deuxième connexion enfichable électrique (100, 100', 605, 605') sont conçus au moins partiellement dans une deuxième couleur, et dans lequel la première et la deuxième couleur sont différentes l'une de l'autre.

4. Agencement d'appareils médicaux (700), comprenant un système de connexion enfichable électrique (500, 600) selon au moins l'une des revendications 1 à 3.

5. Agencement d'appareils médicaux (700) selon la revendication 4, dans lequel une connexion enfichable respective (100, 100', 605, 605') est associée à un tuyau flexible chauffant respectif (792, 792') d'un système de tuyaux flexibles (791) de l'agencement d'appareils médicaux (700), et dans lequel un chauffage (794, 794'), en particulier un chauffage d'un humidificateur de gaz respiratoire, du tuyau flexible chauffant associé (792, 792') est alimenté en courant par la connexion enfichable respective (100, 100', 605, 605').

6. Agencement d'appareils médicaux (700) selon la revendication 5, dans lequel l'élément de réception respectif (120, 120', 620, 620') est connecté de manière non détachable au tuyau flexible chauffant associé (792, 792') par le câble côté réception respectif (128, 128', 628, 628'), et dans lequel l'élément de réception (120, 120', 620, 620'), le câble côté réception (128, 128', 628, 628') et le tuyau flexible chauffant (792, 792') forment ensemble un composant à usage unique (690, 690', 790, 790') de l'agencement d'appareils médicaux (700).
